# EUROPEAN PATENT APPLICATION

(11) **EP 4 801 239 A1**
(43) Date of publication of application: **02.09.2026**
(21) Application number: 24882510.1
(22) Date of filing: 25.10.2024
(51) Int. Cl.: H10K 30/50, C07D 487/22, H10K 30/40, H10K 30/86, H10K 71/12, H10K 85/10, H10K 85/30, H10K 85/50

(54) **METHOD FOR MANUFACTURING PHOTOELECTRIC CONVERSION ELEMENT**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 09.10.2024 JP 2024177310; 23.10.2024 JP 2024186708
(71) Applicant: Canon Kabushiki Kaisha, Tokyo 146-8501 (JP)
(72) Inventor: HAYAKAWA Ai, Tokyo 146-8501 (JP); NISHIDA Tsutomu, Tokyo 146-8501 (JP); OHSAWA Tatsuya, Tokyo 146-8501 (JP); NAKAMURA Nobuhiro, Tokyo 146-8501 (JP)
(74) Representative: TBK
(86) International application number: PCT/JP2024/038158
(87) International publication number: WO 2025/089402

(57) **Abstract**

Provided is a method of producing a photoelectric conversion element having improved adhesiveness between its charge-transporting layer and photoelectric conversion layer. The method is a method of producing a photoelectric conversion element including a first electrode, a second electrode, and a photoelectric conversion layer arranged between the first electrode and the second electrode, the photoelectric conversion layer containing a crystal having a perovskite structure, the method including, in this order, the steps of: performing passivation treatment on a surface of the photoelectric conversion layer; and forming, between a layer formed by the passivation treatment and the first electrode, a charge-transporting layer containing a cyclic conjugated compound in which a plurality of pyrrole rings are bonded by conjugated bonds and an insulating resin.

## Description

### [Technical Field]

The present invention relates to a method of producing a photoelectric conversion element.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage through utilization of a photovoltaic effect in which light energy is absorbed from sunlight to generate an electron and a hole.

Currently, an n-p diode-type silicon (Si) single crystal-based solar cell having a light energy conversion efficiency of more than 20% is widely known, and is actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate, and hence a cost reduction can be expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion characteristic.

For example, in Patent Literature 1, there is a description of a technology including incorporating an organic semiconductor and a polymer compound having a glass transition temperature of 100°C or more into a hole-transporting layer to alleviate its peeling from an electrode. In Non Patent Literature 1, there is a description that conversion efficiency is improved by mixing copper phthalocyanine and a conductive polymer into a hole-transporting layer.

### [Citation List]

### [Patent Literature]

PTL 1: Japanese Patent Laid-Open No. 2018-170382

### [Non Patent Literature]

NPL 1: Q. Hu, et al, Sol. RRL, 2018, 3, 1800264

### [Summary of Invention]

### [Technical Problem]

According to investigations made by the present inventors, it has been found that there is room for improvement in adhesiveness between a charge-transporting layer and a photoelectric conversion layer in each of the photoelectric conversion elements described in Patent Literature 1 and Non Patent Literature 1.

Accordingly, the present invention is directed to providing a method of producing a photoelectric conversion element having improved adhesiveness between its charge-transporting layer and photoelectric conversion layer.

### [Solution to Problem]

In order to solve the above-mentioned problems, the present invention is directed to a method of producing a photoelectric conversion element including a first electrode, a second electrode, and a photoelectric conversion layer arranged between the first electrode and the second electrode, the photoelectric conversion layer containing a crystal having a perovskite structure, the method including, in this order, the steps of: performing passivation treatment on a surface of the photoelectric conversion layer; and forming, between a layer formed by the passivation treatment and the first electrode, a charge-transporting layer containing a cyclic conjugated compound in which a plurality of pyrrole rings are bonded by conjugated bonds and an insulating resin.

### [Advantageous Effects of Invention]

According to the present invention, the method of producing a photoelectric conversion element having improved adhesiveness between its charge-transporting layer and photoelectric conversion layer can be provided. Thus, the characteristics such as photoelectric conversion efficiency of a photoelectric conversion element produced by the production method can be improved.

### [Brief Description of Drawings]

[Fig. 1]

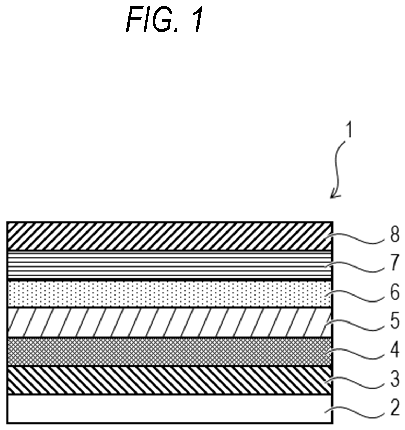

   Fig. 1 is a schematic view of a layer configuration in the thickness direction of a photoelectric conversion element according to a first embodiment of the present invention.
[Fig. 2]

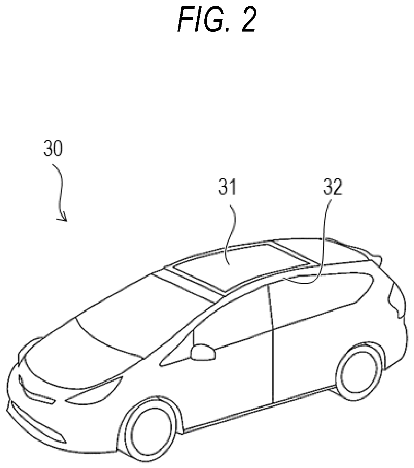

   Fig. 2 is a perspective view for schematically illustrating a moving body including the photoelectric conversion element according to one embodiment of the present invention.
[Fig. 3]

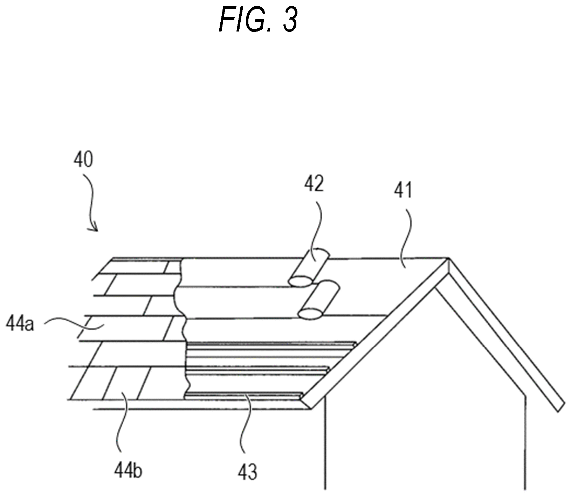

   Fig. 3 is a perspective view for schematically illustrating a building material including the photoelectric conversion element according to one embodiment of the present invention.

### [Description of Embodiments]

### <One Embodiment>

One embodiment is directed to a method of producing a photoelectric conversion element.

A method of producing a photoelectric conversion element of the present invention is
a method of producing a photoelectric conversion element including
a first electrode,
a second electrode, and
a photoelectric conversion layer arranged between the first electrode and the second electrode, the photoelectric conversion layer containing a crystal having a perovskite structure,
the method being characterized by including, in this order, the steps of:
   performing passivation treatment on a surface of the photoelectric conversion layer;
   and forming, between a layer formed by the passivation treatment and the first electrode, a charge-transporting layer containing a cyclic conjugated compound in which a plurality of pyrrole rings are bonded by conjugated bonds and an insulating resin.

As a result of investigations made by the inventors of the present invention, it has been found that adhesiveness between the charge-transporting layer and the photoelectric conversion layer can be improved by the above-mentioned production method. The reason is conceived to be as described below.

The photoelectric conversion layer containing the crystal having a perovskite structure has many crystal defects on a crystal surface. In addition, the charge-transporting layer has a high charge transportation capability, and hence π-conjugated molecules are often used. The above-mentioned crystal defects and π-conjugated molecules each have a part where electrons are biased, and those defects or molecules electrostatically repel each other to reduce adhesiveness. Thus, a gap may be formed between the photoelectric conversion layer and the charge-transporting layer.

When the gap is present, oxygen in the gap causes the deterioration of the photoelectric conversion layer to degrade durability performance, and air acts as a high-resistance layer that hinders charge transfer. Thus, the conversion efficiency of the photoelectric conversion element reduces.

Meanwhile, when the surface of the crystal having a perovskite structure is subjected to the passivation treatment and then the charge-transporting layer is formed, surface crystal defects of the perovskite structure are inactivated by the layer formed by the passivation treatment (hereinafter also referred to as "passivation layer"), and hence electrostatic repulsion is eliminated. At the same time, the charge-transporting layer containing the cyclic conjugated compound in which the plurality of pyrrole rings are bonded by covalent bonds and the insulating resin is formed. Thus, peeling-suppressing and conversion efficiency-improving effects can also be expected.

As a result, it is conceived that the gap between the photoelectric conversion layer and the charge-transporting layer reduces, and hence a photoelectric conversion element having high adhesiveness and improved power generation efficiency is obtained.

### <Step of forming First Electrode and Step of forming Second Electrode>

The method of producing a photoelectric conversion element of the present invention preferably includes the steps of forming the first electrode; and forming the second electrode. In the step of forming the first electrode and the step of forming the second electrode, an appropriate method may be selected in accordance with a material for the first electrode and a material for the second electrode, respectively. Examples of such method include, but are not limited to, a sputtering method, a vacuum vapor deposition method, a vapor phase growth method (CVD method), and a spray pyrolysis deposition method (SPD method). The materials for the first electrode and the second electrode are as described later. When one, or each of both, of the first electrode and the second electrode is a transparent electrode, the thickness of the transparent electrode is preferably 0.03 to 3 µm.

When a solar cell is produced, cutting processing is generally performed for circuit formation between steps. Examples of the cutting processing include mechanical patterning and laser patterning.

### <Modularization Step>

The method of producing a photoelectric conversion element of the present invention may include a modularization step of sealing the element formed up to the electrode. A method for the sealing is, for example, sealing with a resin or sealing with a film containing a resin. Examples of a material used for the sealing include a silazane, a silicone rubber, a resin having a siloxane skeleton, a resin containing fluorine, and glass.

In addition, hairline treatment may be performed on the surface of the sealed element from the viewpoint of the suppression of adhesion between elements occurring during winding in a roll-to-roll system.

### <Step of forming Photoelectric Conversion Layer>

The method of producing a photoelectric conversion element of the present invention preferably includes a step of forming the photoelectric conversion layer containing the crystal having a perovskite structure between the first electrode and the second electrode. The step of forming the photoelectric conversion layer includes a step of applying a liquid containing the material for the photoelectric conversion layer described above. Examples of a method for the application include a spin coating method, a blade coating method, a slit die coating method, a screen printing method, a bar coater method, a casting method, a printing transfer method, a dip-drawing method, an ink jet method, a spray method, and a vacuum vapor deposition method. The method is appropriately selected therefrom in accordance with the properties of a photoelectric conversion layer to be produced, such as thickness control and orientation control.

Annealing treatment may be performed under reduced pressure or under an inert atmosphere (under a nitrogen or argon atmosphere) in order to remove a solvent or a dispersion medium from the applied liquid containing the material for the photoelectric conversion layer. The temperature of the annealing treatment is preferably 40 to 300°C, more preferably 50 to 150°C. The annealing treatment is preferably performed because materials for forming the respective layers may permeate each other at an interface between laminated layers to increase a contact area, and hence a current density can be increased.

### <Step of performing Passivation Treatment>

The method of producing a photoelectric conversion element of the present invention includes a step of performing passivation treatment on the surface of the photoelectric conversion layer on the first electrode side.

The step of performing the passivation treatment may be included in the step of forming the photoelectric conversion layer described above, or the step of performing the passivation treatment may be separately provided after the step of forming the photoelectric conversion layer described above.

The step of performing the passivation treatment includes a step of applying a liquid containing a material for performing the passivation treatment to be described later. Examples of a method for the application include a spin coating method, a blade coating method, a slit die coating method, a screen printing method, a bar coater method, a casting method, a printing transfer method, a dip-drawing method, an ink jet method, a spray method, and a vacuum vapor deposition method. The method is appropriately selected therefrom in accordance with the properties of a photoelectric conversion layer to be produced, such as thickness control and orientation control.

### (Material for performing Passivation Treatment)

In the step of performing the passivation treatment of the present invention, a plurality of materials for performing the passivation treatment each preferably include a compound having at least one group selected from the group consisting of: a hydroxy group; a carbonyl group; a carboxy group; an ester group (ester bond, -CO-O- or -O-CO-); an amino group; an ammonium salt; an ether group (ether bond, -O-); a phosphine oxide group; and a thiol group, and more preferably include a compound having at least one group selected from the group consisting of: an ester group (ester bond, -CO-O- or -O-CO-); and an ammonium salt from the viewpoint of high interaction capability with surface defects of a perovskite crystal and a charge-transporting substance. In the present invention, the above-mentioned compound preferably has a molecular weight of 250 or less from the viewpoint that the compound can interact with surface defects of a micro perovskite crystal.

The material for performing the passivation treatment in the present invention is not particularly limited, and specific examples are given in the following formula (A-1) to the following formula (A-47).

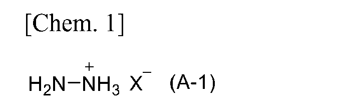

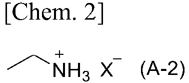

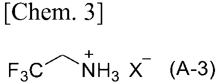

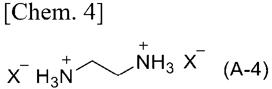

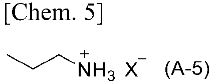

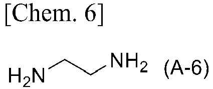

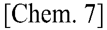

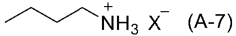

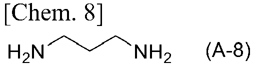

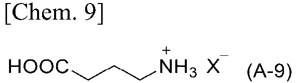

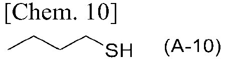

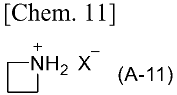

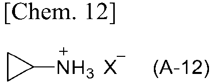

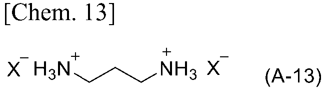

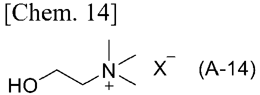

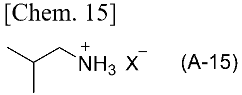

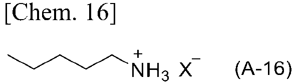

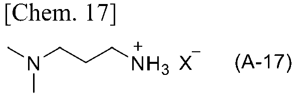

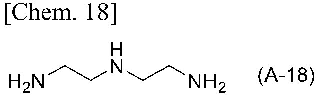

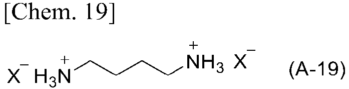

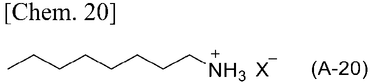

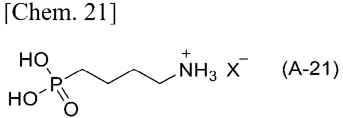

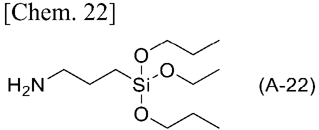

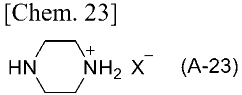

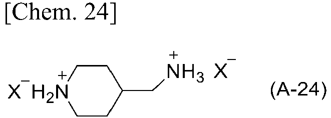

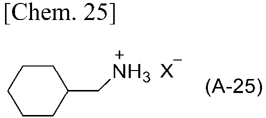

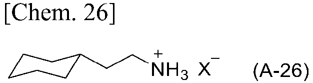

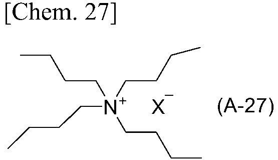

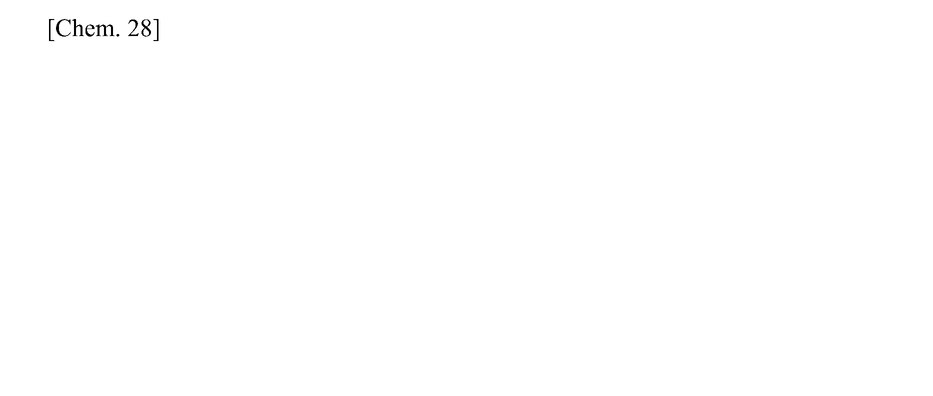

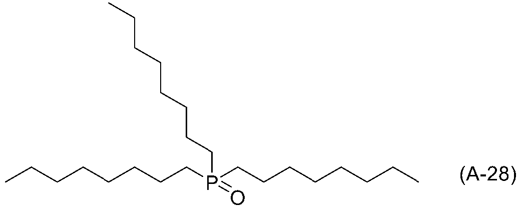

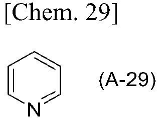

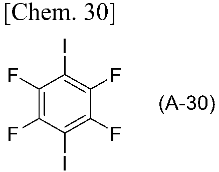

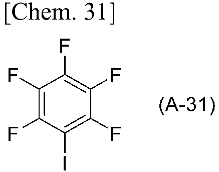

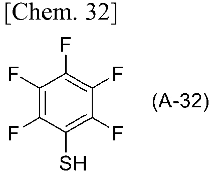

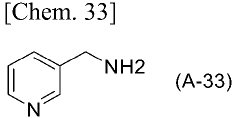

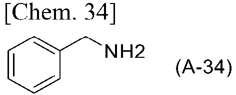

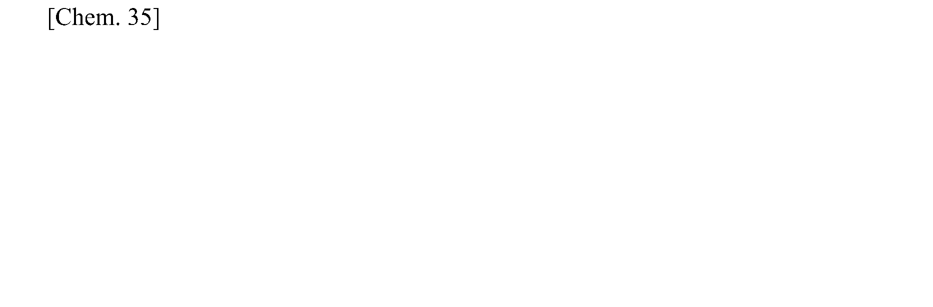

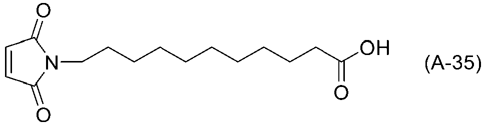

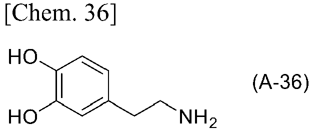

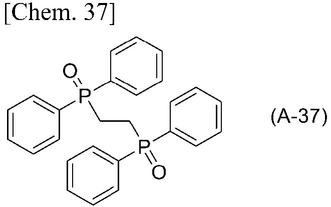

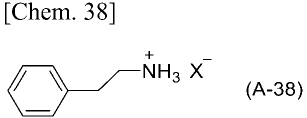

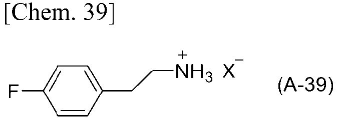

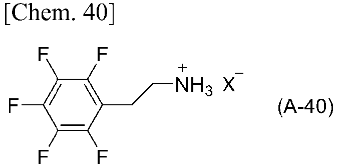

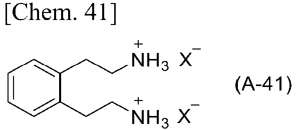

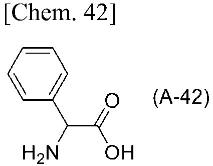

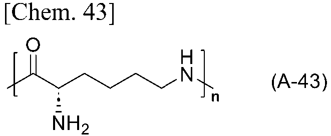

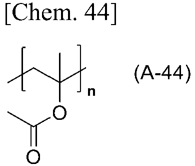

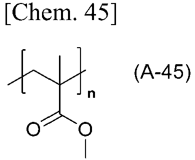

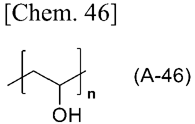

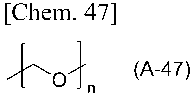

Of those, the compound represented by the formula (A-5), the compound represented by the formula (A-6), the compound represented by the formula (A-10), the compound represented by the formula (A-20), the compound represented by the formula (A-28), the compound represented by the formula (A-31), the compound represented by the formula (A-35), the compound represented by the formula (A-38), the compound represented by the formula (A-39), the compound represented by the formula (A-42), the compound represented by the formula (A-45), the compound represented by the formula (A-46), and the compound represented by the formula (A-47) are desired from the viewpoint of ease of an interaction with crystal defects on the surface of the photoelectric conversion layer.

In the formula (A-1) to the formula (A-41), examples of an anion represented by X⁻ include, but are not limited to, a fluoride ion, a chloride ion, a bromide ion, an iodide ion, a cyanide ion, a nitrate ion, a benzenesulfonate ion, a p-toluenesulfonate ion, a methyl sulfate ion, an ethyl sulfate ion, a propyl sulfate ion, a tetrafluoroborate ion, a tetraphenylborate ion, a benzenesulfinate ion, an acetate ion, a trifluoroacetate ion, a propionacetate ion, a benzoate ion, an oxalate ion, a succinate ion, a malonate ion, an oleate ion, a stearate ion, a citrate ion, a picolinate ion, a monohydrogen diphosphate ion, a dihydrogen diphosphate ion, a pentafluoropropionate ion, a chlorosulfonate ion, a fluorosulfonate ion, a perchlorate anion, a trifluoromethanesulfonyl anion, a bis(trifluoromethanesulfonyl)imide anion, a naphthalenesulfonyl ion, a naphthalenedisulfonate ion, a tristrifluoromethanesulfonylmethide anion, a tetraarylborate anion, and a sulfate anion.

A bromide ion or an iodide ion is particularly preferred as the anion represented by X⁻ from the viewpoint of ease of maintaining a perovskite crystal structure.

In the present invention, the material for performing the passivation treatment preferably includes at least one compound selected from the group consisting of: a compound represented by the following formula (A-38-I); a compound represented by the following formula (A-20-I); and a compound represented by the following formula (A-5-Br).

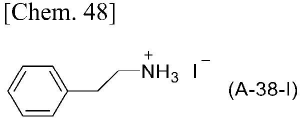

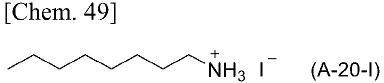

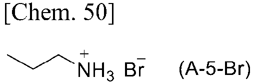

### <Step of forming Charge-transporting Layer>

The method of producing a photoelectric conversion element of the present invention includes a step of forming, between a layer formed by the passivation treatment (passivation layer) and the first electrode, a charge-transporting layer containing a cyclic conjugated compound in which a plurality of pyrrole rings are bonded by conjugated bonds and an insulating resin.

The step of forming the charge-transporting layer is preferably a method including applying a resin solution in which the insulating resin is dissolved. Thus, the insulating resin preferentially penetrates into a gap between perovskite crystal grains with ease.

In addition, examples of the step of forming the charge-transporting layer include: a method including applying the resin solution in which the insulating resin is dissolved after arranging the cyclic conjugated compound in which the plurality of pyrrole rings are bonded by conjugated bonds on the surface of the passivation layer; a method including arranging the cyclic conjugated compound in which the plurality of pyrrole rings are bonded by conjugated bonds after applying the resin solution in which the insulating resin is dissolved on the surface of the passivation layer; or a method including applying a solution in which the cyclic conjugated compound in which the plurality of pyrrole rings are bonded by conjugated bonds is dispersed in the resin solution in which the insulating resin is dissolved onto the surface of the passivation layer.

### (Cyclic Conjugated Compound in which Pyrrole Rings are bonded by Conjugated Bonds)

The cyclic conjugated compound in which the plurality of pyrrole rings are bonded by covalent bonds to be used in the present invention is preferably a porphyrin compound or a phthalocyanine compound, more preferably a phthalocyanine compound from the viewpoint of the spreading of a π-electron cloud serving as the starting point of an interaction. The phthalocyanine compound may have a central element, and examples of the central element include Ga, Cu, Ti, Zn, Si, V, Pb, and Pt. Of those, Ga is preferred from the viewpoint of an electronic interaction with an insulating resin or a passivation material, and the cyclic conjugated compound is more preferably a gallium phthalocyanine compound, particularly preferably a hydroxygallium phthalocyanine compound from the viewpoint of an interaction with a passivation material.

Specific examples of the porphyrin compound in the present invention are given in the following formula (P-1) and the following formula (P-2).

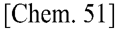

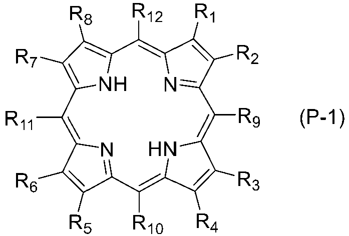

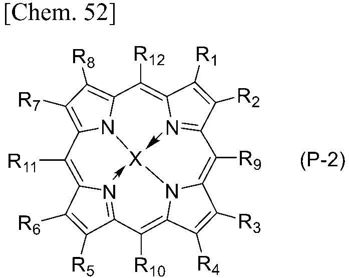

In the formula (P-1) and the formula (P-2), R₁ to R₁₂ each represent an aromatic group that may have a substituent, an organic group that may have a substituent, or the like, and R₁ to R₁₂ may be different from each other.

Specifically, R₁ to R₁₂ each preferably represent a hydrogen atom, a methyl group, an ethyl group, a propyl group, an isopropyl group, a butyl group, an octyloxy group, an octabutoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a tetrasodium sulfonate group, a 4-cumylphenoxy group, a sulfonic acid group, a phenylthio group, and a tert-butyl group. In the formula (P-1) and the formula (P-2), X represents a metal element or an organic element, and specifically, SiCl₂, Cu, Zn, Pd, Pb, Ni, Pt, Co, MnCl, FeCl, VO, and RuCO are preferred.

Specific examples of the phthalocyanine compound in the present invention are given in the following formula (P-3), the following formula (P-4), and the following formula (P-5).

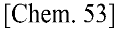

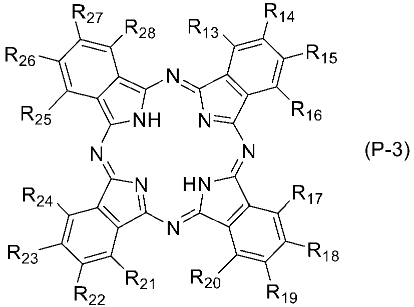

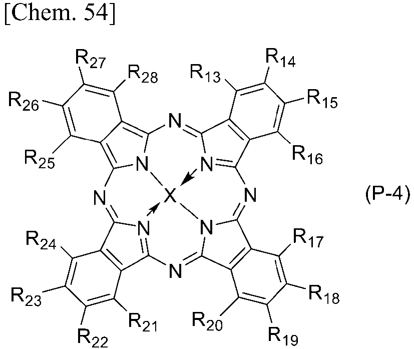

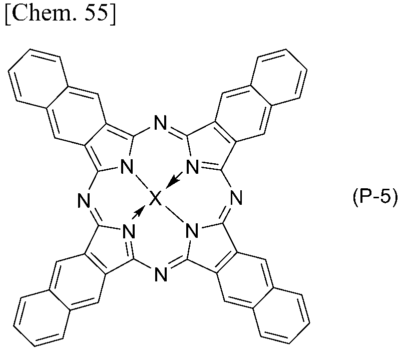

In the formula (P-3) and the formula (P-4), R₁₃ to R₂₈ each represent an aromatic group that may have a substituent or an organic group that may have a substituent, and R₁₃ to R₂₈ may be different from each other. Specifically, a hydrogen atom, a methyl group, an ethyl group, a propyl group, an isopropyl group, a butyl group, an octyloxy group, an octabutoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a tetrasodium sulfonate group, a 4-cumylphenoxy group, a sulfonic acid group, a phenylthio group, and a tert-butyl group are preferred. In the formula (P-3), the formula (P-4), and the formula (P-5), X represents a metal element or an organic element, and specifically, SiCl₂, Cu, Zn, Pd, Pb, Ni, Pt, Co, MnCl, FeCl, VO, and RuCO are preferred.

### (Insulating Resin)

Specific examples of the insulating resin include a polyacetal resin, an acrylic resin, a polyarylate resin, a polycarbonate resin, a polyvinyl acetate resin, a polyester resin, a polyamide resin, a polyurethane resin, and a polystyrene resin.

In the present invention, the glass transition temperature of the insulating resin is preferably 95°C or less. When the glass transition temperature falls within this range, the insulating resin is easily brought into close contact with a charge-transporting material (cyclic conjugated compound in which pyrrole rings are bonded by conjugated bonds), and hence a more effective charge distribution can be formed. The glass transition temperature may be determined with a differential scanning calorimeter (DSC).

In the present invention, the insulating resin is preferably a polyvinyl acetal resin or a polyvinyl butyral resin. The insulating resin is easily brought into close contact with the charge-transporting material (cyclic conjugated compound in which pyrrole rings are bonded by conjugated bonds), and hence a more effective charge distribution can be formed.

In the present invention, the photoelectric conversion element may include a second charge-transporting layer between the first electrode and the charge-transporting layer. When the photoelectric conversion element includes the second charge-transporting layer, the transfer of a carrier to an electrode may be facilitated.

The effects of the present invention can be achieved when the respective configurations synergistically exhibit effects on each other through such mechanism as described above.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of a constituent element gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and determining the element distribution of a specific element. The analysis of each layer may be performed by peeling and removing a layer from a completed photoelectric conversion element to expose the layer to be analyzed. In the present invention, for the quantification of a volume ratio, the area ratio of an exposed surface or a cross section is used as the volume ratio of the layer.

In the method of producing a photoelectric conversion element of the present invention, the photoelectric conversion element includes a first electrode, a second electrode, and a photoelectric conversion layer arranged between the first electrode and the second electrode, the photoelectric conversion layer containing a crystal having a perovskite structure. Fig. 1 is a sectional view for schematically illustrating the configuration of the photoelectric conversion element according to one embodiment of the present invention. A photoelectric conversion element 1 includes a substrate 2, and a second electrode 3, an electron-transporting layer 4, a photoelectric conversion layer 5, a passivation layer 6, a charge-transporting layer 7, and a first electrode 8 arranged thereon. One of the first electrode 8 and the second electrode 3 is an anode, and the other is a cathode. A current can be extracted by connecting the first electrode 8 and the second electrode 3 with an external circuit.

The photoelectric conversion layer 5 is excited by light that has entered the layer through the substrate 2, the second electrode 3, and the electron-transporting layer 4, or the first electrode 8, the charge-transporting layer 7, and the passivation layer 6 to generate an electron or a hole. That is, the photoelectric conversion layer 5 generates a current between the first electrode 8 and the second electrode 3. The electron-transporting layer 4 is a layer arranged between the photoelectric conversion layer 5, and the two electrodes (the second electrode 3 and the first electrode 8), and may not be formed in some cases. A form in which the plurality of electron-transporting layers 4 and photoelectric conversion layers 5 are laminated may be adopted. Such form may also be referred to as "tandem structure." The respective members are described below. In addition, the photoelectric conversion element may be produced in the order of the first electrode 8, the charge-transporting layer 7, the passivation layer 6, the photoelectric conversion layer 5, the electron-transporting layer 4, and the second electrode 3 on the substrate 2.

### [Photoelectric Conversion Element]

The present invention is directed to a method of producing a photoelectric conversion element. The photoelectric conversion element of the present invention is characterized by including: the first electrode; the second electrode; the photoelectric conversion layer arranged between the first electrode and the second electrode, the photoelectric conversion layer containing the crystal having a perovskite structure; and the charge-transporting layer between the photoelectric conversion layer and the first electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a perovskite crystal in its photoelectric conversion layer.

Examples of a method of forming each of the layers including the photoelectric conversion layer and charge-transporting layer of the photoelectric conversion element of the present invention include a coating method and a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with the respective layers.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 8 side, an opaque material may be used as the substrate 2, and when light is taken in from the second electrode 3 side, the substrate 2 is formed of a transparent material.

### [Electrode]

The photoelectric conversion element of the present invention includes the first electrode and the second electrode. A material for the first electrode 8 or the second electrode 3 is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesiumindium mixture; an aluminum-lithium alloy; an Al/Al₂O₃ mixture; and an Al/LiF mixture.

Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof.

At least one electrode of the first electrode 8 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 8 is on the light incident side, the second electrode 3 and the substrate 2 may be a transparent electrode and a reflective layer, respectively. The electrode may be a patterned electrode.

### [Photoelectric Conversion Layer]

The photoelectric conversion element of the present invention includes the photoelectric conversion layer arranged between the first electrode and the second electrode, the photoelectric conversion layer containing the crystal having a perovskite structure. The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚNₘHₙ ("p", "m", and "n" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer.

The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

The crystal having a two-dimensional perovskite or 2.5-dimensional perovskite structure is preferably represented by each of the following general formulae [2] to [4]. "n" represented in each of the following general formulae represents a positive integer.

R'₂Aₙ₋₁BₙX₃ₙ₊₁ [2]

R"Aₙ₋₁BₙX₃ₙ₊₁ [3]

R‴AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule or a metal that may have a substituent. Specifically, ethylammonium, propylammonium, n-butylammonium, n-hexylammonium, n-octylammonium, 1,6-hexanediammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbitals. Those metal atoms may be used alone or in combination thereof.

X in each of the general formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence its application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I_{16,} (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred. The A site, B site, or X site of each of the general formulae may be adjusted to be deficient or excessive in accordance with purposes, and the combinations of x1 to x5 may be changed in accordance with purposes. The combinations of x1 to x5 are, for example, as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiopheneethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

An organic-inorganic perovskite compound to be used in the present invention is preferably a crystalline semiconductor. The term "crystalline semiconductor" means a semiconductor that enables the measurement of an X-ray scattering intensity distribution to detect a scattering peak. When the organic-inorganic perovskite compound is the crystalline semiconductor, the mobility of an electron in the organic-inorganic perovskite compound increases, and the photoelectric conversion efficiency of the photoelectric conversion element is improved.

In addition, the photoelectric conversion layer according to the present invention may include a material except the crystal having an organic-inorganic perovskite structure to the extent that the photoelectric conversion efficiency and the charge transportability are not impaired.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

In the present invention, the surface roughness Ra of the crystal having a perovskite structure of the photoelectric conversion layer is preferably 10 to 200 nm. When the surface roughness Ra is 10 nm or more, the recombination-suppressing effect of the insulating resin is more easily exhibited. In addition, when the surface roughness Ra is larger than 200 nm, the charge-transporting particle is liable to penetrate to the second electrode side of the photoelectric conversion layer, and the recombination-suppressing effect may reduce.

### [Passivation Layer]

In the photoelectric conversion element of the present invention, the passivation layer is formed on the surface of the photoelectric conversion layer on the first electrode side. The passivation layer contains a passivation material. In the photoelectric conversion element of the present invention, the passivation material is preferably arranged between the crystals each having a perovskite structure of the photoelectric conversion layer. The respective items of the passivation material are as described above.

### [Charge-transporting Layer]

In the photoelectric conversion element of the present invention, the charge-transporting layer is arranged between the photoelectric conversion layer and the first electrode, and the charge-transporting layer is formed of a cyclic conjugated compound in which pyrrole rings are bonded by conjugated bonds and an insulating resin on the surface of the passivation layer. In the photoelectric conversion element of the present invention, the photoelectric conversion layer and the passivation layer are preferably completely covered. The respective items, such as the cyclic conjugated compound in which the pyrrole rings are bonded by conjugated bonds and the insulating resin, are as described above.

In the present invention, the charge-transporting layer contains the cyclic conjugated compound in which the pyrrole rings are bonded by conjugated bonds and the insulating resin, and the volume of the cyclic conjugated compound in which the pyrrole rings are bonded by conjugated bonds in the charge-transporting layer is preferably 5 to 30 times with respect to the volume of the insulating resin in the charge-transporting layer. The insulating resin has a volume resistivity of 10⁸ Ω·cm or more. In the present invention, during charge transportation, the content mass of the cyclic conjugated compound in which the pyrrole rings are bonded by conjugated bonds is preferably 5 to 30 times with respect to the content mass of the insulating resin.

The thickness of the charge-transporting layer is preferably 1 to 1,000 nm, more preferably 5 to 500 nm, particularly preferably 10 to 200 nm.

The charge-transporting layer may be formed by preparing a coating liquid for a charge-transporting layer containing the above-mentioned respective materials and a solvent, forming a coating film of the coating liquid on the passivation layer, and drying the coating film. Examples of the solvent to be used for the coating liquid include an alcohol-based solvent, a ketone-based solvent, an ether-based solvent, an ester-based solvent, and an aromatic hydrocarbon-based solvent. Of those solvents, an alcohol-based solvent or an aromatic hydrocarbon-based solvent is preferred.

### [Second Charge-transporting Layer]

In the present invention, the photoelectric conversion element 1 may further include the second charge-transporting layer between the charge-transporting layer 7 and the first electrode 8 from the viewpoint of the compatibility of a film of the charge-transporting layer 7.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. The compound preferably has an aromatic ring from the viewpoint of the compatibility of a film interface, and Spiro-OMeTAD, PTAA, or a phthalocyanine compound is particularly preferred.

In addition, the second charge-transporting layer may contain a dopant as an additive in order to improve its charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element of the present invention, the electron-transporting layer 4 may be arranged between the second electrode 3 and the photoelectric conversion layer 5 as illustrated in Fig. 1.

A material for the electron-transporting layer 4 is not particularly limited, and examples thereof include an N-type conductive polymer, an N-type lowmolecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide. In particular, tin oxide may be obtained through the reaction of tin(II) chloride, tin(IV) chloride, tin(II) chloride dihydrate, or tin(IV) chloride pentahydrate.

A preferred lower limit of the thickness of the electron-transporting layer 4 is 1 nm, and a preferred upper limit thereof is 2,000 nm. When the thickness of the electron-transporting layer 4 is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material.

### [Photoelectric Conversion Apparatus]

A photoelectric conversion apparatus of the present invention includes the above-mentioned photoelectric conversion element. The photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." Photoelectric conversion elements having different absorption wavelengths may be laminated as the photoelectric conversion elements to increase an output voltage.

In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function of, for example, maintaining or increasing the amount of incident light, a surface layer to which water or dirt is hard to adhere, or a function of collecting or guiding light may be added.

### [Moving Body]

A moving body of the present invention includes the above-mentioned photoelectric conversion element. Fig. 2 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

A building material of the present invention includes the above-mentioned photoelectric conversion element. Fig. 3 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

In addition to the application examples described above, the following application examples are given: portable devices, such as a calculator, a sensor, and a small solar panel; wearable devices, such as a glasses-type terminal, a watch-type terminal, and a portable medical device; sheet structures supported by a plurality of frames, such as a tent, a plastic greenhouse, and a loading platform of a truck; and structures to be used by being fixed, such as a road surface panel, a floating panel, a building material utilizing the flexibility of a substrate, a wall-type building material, a glass-type building material, and a mega solar panel.

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### (Example 1)

### <Production of Charge-transporting Substance 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide chlorogallium phthalocyanine in a yield of 71%.

### Step (2)

4.65 Parts of the chlorogallium phthalocyanine was dissolved in 139.5 parts of concentrated sulfuric acid at a temperature of 10°C, and the solution was dropped into 620 parts of ice water under stirring so that its particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times.

Finally, the filter residue was freeze-dried to provide hydroxygallium phthalocyanine (hydrous hydroxygallium phthalocyanine) having a solid content of 23 mass% in a yield of 71%. The hydroxygallium phthalocyanine was dried with a hyper-dry dryer (product name: HD-06R, frequency (oscillatory frequency): 2,455 MHz±15 MHz, manufactured by Biocon (Japan) Ltd.). Thus, hydroxygallium phthalocyanine (OHGaPc) (crystal) having a water content of 1.0 mass% or less was obtained.

### Step (3)

5 Parts of the hydroxygallium phthalocyanine was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) containing 5 parts of glass beads, followed by filtration and drying to provide a charge-transporting substance 1 (specific gravity: 1.6).

### <Production of Resin Solution 1>

1.0 Gram of polyvinyl butyral (product name: BM-2, manufactured by Sekisui Chemical Co., Ltd., specific gravity: 1.6) was dissolved in 19 g of 2-propanol by stirring for 24 hours to provide a resin solution 1.

### <Production of Passivation Solution 1>

2.5 Milligrams of the compound (A-5) using a bromide ion for X⁻ (2-phenylethylamine hydroiodide, PEAI, Tokyo Chemical Industry Co., Ltd.) was dissolved in 1 ml of 2-propanol to provide a passivation solution 1.

### [Formation of Electron-transporting Layer]

A 25 mm×25 mm square glass substrate with ITO was washed, and a tin(II) oxide colloidal solution (15% water dispersion, manufactured by Alfa Aesar) diluted fivefold was applied thereonto by spin coating, followed by heating at 150°C for 30 minutes to form an electron-transporting layer as a thin film having a thickness of 16 nm.

### [Formation of Photoelectric Conversion Layer]

0.487 Gram of lead bromide, 1.034 g of formamidinium iodide, 2.903 g of lead iodide, and 0.139 g of methylammonium bromide were dissolved in 4.25 g of N,N-dimethylformamide and 1.216 g of dimethyl sulfoxide, and the solution was stirred for 1 hour (solution 1). Further, 0.100 g of cesium iodide was dissolved in 0.285 g of dimethyl sulfoxide, and the solution was stirred for 1 hour (solution 2). After that, the cesium iodide solution (solution 2) was added to the solution 1 to prepare a photoelectric conversion layer coating liquid. The coating liquid was applied onto the electron-transporting layer by spin coating in accordance with a poor solvent method to form a photoelectric conversion layer formed of Cs_{0.05}(FA_{0.83}MA_{0.17})_{0.95}Pb(I_{0.83}Br_{0.17})₃, the layer having a thickness of 600 nm and a surface roughness Ra of 13 nm.

### [Formation of Passivation Layer]

The passivation solution was applied onto the photoelectric conversion layer by spin coating to form a passivation layer having a thickness of 10 nm.

### [Formation of Charge-transporting Layer]

0.1 Gram of the charge-transporting substance 1 and 0.01 g of a calixarene compound (Japanese Patent Laid-Open No. 2003-207913) were mixed with 10.6 g of 2-propanol, and 11 g of beads (zirconia beads, Torayceram (trademark) zirconia beads, 0.3 mm) were loaded into the mixture, followed by dispersion with a paint shaker (manufactured by Toyo Seiki Seisaku-sho, Ltd.) for 7 hours. After that, 0.2 g of the resin solution 1 was added thereto, and the mixture was dispersed with the paint shaker again for 6 hours to prepare a charge-transporting layer solution. The charge-transporting layer solution was applied onto the passivation layer by spin coating to form a charge-transporting layer having a thickness of 160 nm.

### [Introduction of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithium bis(trifluoromethanesulfonyl)imide in 0.3 g of acetonitrile and 60 µL of 4-tert-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a material solution for a second charge-transporting layer. The material solution was applied onto the above-mentioned charge-transporting layer by a spin coating method to form a second charge-transporting layer having a thickness of 100 nm.

### [Formation of First Electrode]

Ten gold electrodes each having a thickness of 80 nm and an area of 0.09 cm² were formed on the second charge-transporting layer by a vacuum vapor deposition method. Thus, a photoelectric conversion element was obtained.

### [Analysis of Amount of Compound]

The electrode surface of the photoelectric conversion element was peeled off so that the surface of the charge-transporting layer was exposed. The surface of the charge-transporting layer was wiped with a cotton swab or the like with a solvent, dissolved in deuterated sulfuric acid, and subjected to ¹H-NMR measurement (apparatus: AVANCE III 500, manufactured by BRUKER). In addition, the presence of a compound was recognized by subjecting the peeled-off charge-transporting layer components to mass and structure analyses through GPC and MALDI-TOF-MS, IR, gas chromatography, and elemental analysis, such as XPS or EDX.

In addition, the thickness of the photoelectric conversion element was determined with a scanning electron microscope (apparatus: SmartSEM, manufactured by Carl Zeiss Co., Ltd.) after the cutting of the photoelectric conversion element and the fixing of the sample to a tilted sample stage.

### (Example 2)

A photoelectric conversion element is obtained in the same manner as in Example 1 except that the passivation material for the passivation solution 1 is changed to the material described in the compound (A-45).

### (Example 3)

A photoelectric conversion element is obtained in the same manner as in Example 1 except that the charge-transporting substance 1 is changed to the following formula (Pc-1).

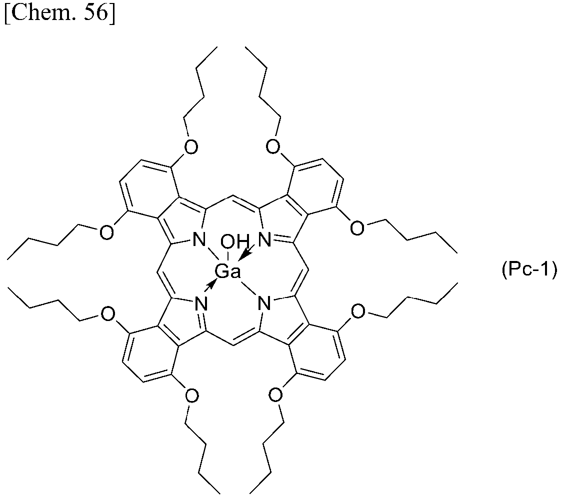

### (Example 4)

A photoelectric conversion element is obtained in the same manner as in Example 3 except that the passivation material for the passivation solution 1 is changed to the material described in the compound (A-20).

### (Example 5)

A photoelectric conversion element is obtained in the same manner as in Example 3 except that the passivation material for the passivation solution 1 is changed to the material described in the compound (A-5). A bromide ion is used for X⁻ in the compound (A-5).

### (Example 6)

A photoelectric conversion element is obtained in the same manner as in Example 2 except that the charge-transporting substance 1 is changed to the following formula (Pc-1).

### (Example 7)

A photoelectric conversion element is obtained in the same manner as in Example 1 except that the charge-transporting substance 1 is changed to chlorogallium phthalocyanine.

### (Example 8)

A photoelectric conversion element is obtained in the same manner as in Example 1 except that the passivation material for the passivation solution 1 is changed to the material described in the compound (A-46).

### (Example 9)

A photoelectric conversion element is obtained in the same manner as in Example 1 except that the passivation material for the passivation solution 1 is changed to the material described in the compound (A-35).

### (Example 10)

A photoelectric conversion element is obtained in the same manner as in Example 1 except that the passivation material for the passivation solution 1 is changed to the material described in the compound (A-42).

### (Example 11)

A photoelectric conversion element is obtained in the same manner as in Example 1 except that the passivation material for the passivation solution 1 is changed to the material described in the compound (A-6).

### (Example 12)

A photoelectric conversion element is obtained in the same manner as in Example 1 except that the ratio of the volume of the charge-transporting substance 1 to the volume of the insulating resin is set to 5.

### (Example 13)

A photoelectric conversion element is obtained in the same manner as in Example 1 except that the charge-transporting substance 1 is changed to copper phthalocyanine.

### (Example 14)

A photoelectric conversion element is obtained in the same manner as in Example 1 except that the passivation material for the passivation solution 1 is changed to the material described in the compound (A-10).

### (Example 15)

A photoelectric conversion element is obtained in the same manner as in Example 1 except that the passivation material for the passivation solution 1 is changed to the material described in the compound (A-39). An iodide ion is used for X⁻ in the compound (A-39).

### (Example 16)

A photoelectric conversion element is obtained in the same manner as in Example 1 except that the passivation material for the passivation solution 1 is changed to the material described in the compound (A-28).

### (Example 17)

A photoelectric conversion element is obtained in the same manner as in Example 1 except that the passivation material for the passivation solution 1 is changed to the material described in the compound (A-47).

### (Example 18)

A photoelectric conversion element is obtained in the same manner as in Example 1 except that the insulating resin is changed to polymethyl methacrylate (PMMA, manufactured by Sigma-Aldrich Co. LLC, glass transition temperature: 100°C).

### (Example 19)

A photoelectric conversion element is obtained in the same manner as in Example 2 except that the ratio of the volume of the charge-transporting substance 1 to the volume of the insulating resin is set to 1.

### (Example 20)

A photoelectric conversion element is obtained in the same manner as in Example 1 except that the charge-transporting substance 1 is changed to tetraphenylporphyrin (TPP).

### (Example 21)

A photoelectric conversion element is obtained in the same manner as in Example 1 except that the ratio of the volume of the charge-transporting substance 1 to the volume of the insulating resin is set to 30.

### (Example 22)

A photoelectric conversion element is obtained in the same manner as in Example 1 except that the passivation material for the passivation solution 1 is changed to the material described in the compound (A-31).

### (Example 23)

A photoelectric conversion element is obtained in the same manner as in Example 2 except that the ratio of the volume of the charge-transporting substance 1 to the volume of the insulating resin is set to 40.

### (Comparative Example 1)

A photoelectric conversion element is obtained in the same manner as in Example 12 except that the charge-transporting substance 1 is changed to Spiro-OMeTAD.

### (Comparative Example 2)

A photoelectric conversion element is obtained in the same manner as in Example 3 except that poly(3-hexylthiophene-2,5-diyl) (P3HT) is used as a conductive resin instead of the insulating resin.

### (Comparative Example 3)

A photoelectric conversion element is obtained in the same manner as in Example 3 except that the passivation solution is not used.

### [Evaluation]

### (Gap Length)

The photoelectric conversion element produced in Example 1 was cut and fixed to a sample stage, and then an observation image of the cut surface was obtained with a scanning electron microscope (apparatus: SmartSEM, Carl Zeiss Co., Ltd.). The height of a gap between the surface of the photoelectric conversion layer and the charge-transporting layer in the observation image was measured and used as a gap length. The gap length was measured at five sites in the image, and the average of the measured values was adopted as the representative value of the gap length. The results are shown in Table 2. The direction of the height of the gap is a direction perpendicular to the surface of the photoelectric conversion layer.

### [Photoelectric Conversion Efficiency]

A power supply (236 model, manufactured by Keithley Instruments, LLC) is connected between the electrodes of the photoelectric conversion element produced in Example 1, and its photoelectric conversion efficiency is measured by: irradiating the element with constant light through use of a solar simulator (manufactured by Yamashita Denso Corporation) having an intensity of 114 mW/cm²; and measuring the generated current and voltage. In addition, each of the ten electrodes for each photoelectric conversion element is subjected to the measurement, and the average of the measured values is adopted as the representative value of the photoelectric conversion element. The results are shown in Table 2.

In Table 2, the photoelectric conversion efficiency in Example 1 is set to 100, and a ratio thereto is shown as the conversion efficiency of each photoelectric conversion element. In Table 2, the term "mass" refers to the ratio of the content mass of the cyclic conjugated compound to the content mass of the insulating resin in the charge-transporting layer.

### [Table 2]

**Table 2**

| Example | Charge-transporting layer | | | Passivation layer | | Adhesiveness | Element characteristic |
|---|---|---|---|---|---|---|---|
| | Cyclic conjugated compound in which a plurality of pyrrole rings are bonded by conjugated bonds | Resin | Mass *relative to resin | Compound | Anion | Gap length [nm] | Photoelectric conversion efficiency [%] |
| Example 1 | Hydroxygallium phthalocyanine | BM-2 | 10 | A-38 | Iodine | 0 | 100.0 |
| Example 2 | Hydroxygallium phthalocyanine | BM-2 | 10 | A-45 | - | 0 | 97.6 |
| Example 3 | (Pc-1) | BM-2 | 10 | A-38 | Iodine | 0 | 97.0 |
| Example 4 | (Pc-1) | BM-2 | 10 | A-20 | Iodine | 0 | 96.4 |
| Example 5 | (Pc-1) | BM-2 | 10 | A-5 | Bromine | 0 | 94.0 |
| Example 6 | (Pc-1) | BM-2 | 10 | A-45 | - | 0 | 95.8 |
| Example 7 | Chlorogallium phthalocyanine | BM-2 | 10 | A-38 | Iodine | 2 | 92.8 |
| Example 8 | Hydroxygallium phthalocyanine | BM-2 | 10 | A-46 | - | 2 | 88.6 |
| Example 9 | Hydroxygallium phthalocyanine | BM-2 | 10 | A-35 | - | 2 | 89.2 |
| Example 10 | Hydroxygallium phthalocyanine | BM-2 | 10 | A-42 | - | 3 | 90.4 |
| Example 11 | Hydroxygallium phthalocyanine | BM-2 | 10 | A-6 | - | 3 | 96.4 |
| Example 12 | Hydroxygallium phthalocyanine | BM-2 | 5 | A-38 | Iodine | 4 | 80.8 |
| Example 13 | Copper phthalocyanine | BM-2 | 10 | A-38 | Iodine | 5 | 77.8 |
| Example 14 | Hydroxygallium phthalocyanine | BM-2 | 10 | A-10 | - | 5 | 84.4 |
| Example 15 | Hydroxygallium phthalocyanine | BM-2 | 10 | A-39 | Iodine | 5 | 82.6 |
| Example 16 | Hydroxygallium phthalocyanine | BM-2 | 10 | A-28 | - | 6 | 87.4 |
| Example 17 | Hydroxygallium phthalocyanine | BM-2 | 10 | A-47 | - | 8 | 86.8 |
| Example 18 | Hydroxygallium phthalocyanine | PMMA | 10 | A-38 | Iodine | 8 | 76.6 |
| Example 19 | Hydroxygallium phthalocyanine | BM-2 | 1 | A-45 | - | 9 | 71.9 |
| Example 20 | TPP | BM-2 | 10 | A-38 | Iodine | 10 | 73.1 |
| Example 21 | Hydroxygallium phthalocyanine | BM-2 | 30 | A-38 | Iodine | 12 | 91.0 |
| Example 22 | Hydroxygallium phthalocyanine | BM-2 | 10 | A-31 | - | 15 | 80.8 |
| Example 23 | Hydroxygallium phthalocyanine | BM-2 | 40 | A-45 | - | 16 | 73.1 |
| Comparative Example 1 | Spiro-OMeTAD | BM-2 | 5 | A-38 | Iodine | 45 | 53.3 |
| Comparative Example 2 | (Pc-1) | P3HT | 10 | A-38 | Iodine | 48 | 70.1 |
| Comparative Example 3 | (Pc-1) | BM-2 | 10 | - | - | 60 | 65.9 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-177310 filed on October 9, 2024, and Japanese Patent Application No. 2024-186708 filed on October 23, 2024, and the entire contents thereof are incorporated herein by reference.

### [Reference Signs List]

1 photoelectric conversion element
2 substrate
3 second electrode
4 electron-transporting layer
5 photoelectric conversion layer
6 passivation layer
7 charge-transporting layer
8 first electrode
30 moving body
31, 41 photoelectric conversion element
32 body
40 building material
42 protective member
43 heat dissipation member
44a, 44b exterior

## Claims

1. A method of producing a photoelectric conversion element including a first electrode, a second electrode, and a photoelectric conversion layer arranged between the first electrode and the second electrode, the photoelectric conversion layer containing a crystal having a perovskite structure, the method comprising, in this order, the steps of:
performing passivation treatment on a surface of the photoelectric conversion layer; and
forming, between a layer formed by the passivation treatment and the first electrode, a charge-transporting layer containing a cyclic conjugated compound in which a plurality of pyrrole rings are bonded by conjugated bonds and an insulating resin.

2. The method of producing a photoelectric conversion element according to claim 1, wherein the insulating resin has a glass transition temperature of 95°C or less.

3. The method of producing a photoelectric conversion element according to claim 1 or 2, wherein, in the charge-transporting layer, a content mass of the cyclic conjugated compound is 5 to 30 times with respect to a content mass of the insulating resin.

4. The method of producing a photoelectric conversion element according to any one of claims 1 to 3, wherein the cyclic conjugated compound is a phthalocyanine compound.

5. The method of producing a photoelectric conversion element according to any one of claims 1 to 4, wherein the cyclic conjugated compound is a gallium phthalocyanine compound.

6. The method of producing a photoelectric conversion element according to any one of claims 1 to 5, wherein the cyclic conjugated compound is a hydroxygallium phthalocyanine compound.

7. The method of producing a photoelectric conversion element according to any one of claims 1 to 6, wherein, in the step of performing the passivation treatment, a material for performing the passivation treatment includes a compound having at least one group selected from the group consisting of: a hydroxy group; a carbonyl group; a carboxy group; an ester group; an amino group; an ammonium salt; an ether group; a phosphine oxide group; and a thiol group.

8. The method of producing a photoelectric conversion element according to any one of claims 1 to 7, wherein, in the step of performing the passivation treatment, a material for performing the passivation treatment includes a compound having at least one group selected from the group consisting of: an ester group; and an ammonium salt.

9. The method of producing a photoelectric conversion element according to claim 7 or 8, wherein the compound has a molecular weight of 250 or less.

10. The method of producing a photoelectric conversion element according to any one of claims 1 to 9, wherein a material for performing the passivation treatment includes at least one compound selected from the group consisting of: a compound represented by the following formula (A-38-I); a compound represented by the following formula (A-20-I); and a compound represented by the following formula (A-5-Br).
